# EUROPEAN PATENT APPLICATION

(11) **EP 3 495 054 A2**
(43) Date of publication of application: **12.06.2019**
(21) Application number: 18207458.3
(22) Date of filing: 21.11.2018
(51) Int. Cl.: B06B 1/02

(54) **ULTRASOUND IMAGING PROBE, MANUFACTURING METHOD THEREOF, AND ULTRASONIC IMAGING DEVICE**

(30) Priority: 04.12.2017 JP 2017232365
(71) Applicant: Hitachi, Ltd., Tokyo 100-8280 (JP)
(72) Inventor: TAKEZAKI, Taiichi, Tokyo, Aichi 100-8280 (JP); MACHIDA, Shuntaro, Tokyo, Aichi 100-8280 (JP); HASEGAWA, Hiroaki, Tokyo, Aichi 100-8280 (JP); TANAKA, Tomohiko, Tokyo, Aichi 100-8280 (JP); IMAI, Ryo, Tokyo, 100-8280 (JP); SEO, Yoshiho, Tokyo, 100-8280 (JP); MATSUDA, Takahiro, Tokyo, 100-8280 (JP); ONOE, Shinsuke, Tokyo, 100-8280 (JP)
(74) Representative: Strehl Schübel-Hopf & Partner

(57) **Abstract**

An ultrasound imaging probe capable of securing an assembly accuracy of and improving a resolution performance of an obtained image is provided.

A photoacoustic catheter 5 includes: a silicon substrate 5a which includes an ultrasonic transducer 5b for detecting an ultrasonic wave formed thereon and a through hole 5c passing through front and rear surfaces; an optical fiber 5d which oscillates a laser 7; a lens 5e which condenses the laser 7 and is arranged within the through hole 5c; a tubular housing 5f; a glass cover 5i which covers the lens 5e; and a resin 5g which fills a gap 10 between the through hole 5c and the lens 5e. Further, the silicon substrate 5a and the optical fiber 5d are fixed to a part of the housing 5f in the housing 5f.

## Description

### Technical Field

The present invention relates to an ultrasound imaging probe using an ultrasonic transducer, a manufacturing method thereof, and an ultrasonic imaging device.

### Background Art

In an ultrasonic imaging device in which a vascular catheter used in a medical field is attached, for example, a crimped state or the like of the stent and the vascular wall is imaged by radiating an ultrasonic wave on an inspection object part of a blood vessel to detect the ultrasonic wave which is reflected therefrom.

A forward-looking member intended to image the front side of the catheter is used to perform the ultrasonic wave inspection on the inside of the blood vessel. A forward-looking catheter is used to mainly image a portion (thrombus) of which the blood vessel is occluded by a tumor or the like. For this reason, in the forward-looking catheter, it is requested that the inside of the blood vessel is imaged with a large field of view. In addition, the length of the thrombus may reach several centimeters, and it is requested to image the deep portion. In the conventional method of radiating the ultrasonic wave and detecting the ultrasonic wave reflected therefrom, the deep portion can be imaged, but it is difficult to secure a visual field for imaging the inside of the entire blood vessel including the thrombus.

In this regard, a photoacoustic catheter which captures an image by radiating laser on the inside of the entire blood vessel including the thrombus with a wide angle and detecting the ultrasonic wave output therefrom by an ultrasonic transducer is effective.

For example, JP-A-2013-99589 (PTL 1) discloses a structure of an imaging probe in which a hole is formed in a transducer itself, and a lens is arranged in the hole.

### Citation List

### Patent Literature

PTL 1: JP-A-2013-99589

### Summary of Invention

### Technical Problem

In the photoacoustic catheter, it is necessary to arrange and fix an acoustic element such as an ultrasonic transducer and an optical element such as an optical fiber or a lens with an accuracy of 100 µm or less. In the assembly of the above-described vascular catheter which transmits and receives an ultrasonic wave, an element component of 1 mm or less is manually positioned under a microscope by a visual observation. In the assembly method, it is difficult to assemble the photoacoustic catheter, and there is a problem to establish a technology which can secure an assembly accuracy of the photoacoustic catheter.

Incidentally, the imaging probe described in PTL 1 does not use a substrate (silicon substrate) in which the element of the ultrasonic transducer is formed, and the assembly method of the imaging probe cannot be applied to the component mounting of the photoacoustic catheter.

An object of the invention is to provide a technology which can secure an assembly accuracy of an ultrasound imaging probe to improve a resolution performance of an obtained image.

The above object and novel features of the invention will become apparent from the description of this specification and the accompanying drawings.

### Solution to Problem

An outline of representative features in embodiments disclosed in this application will be described in brief as follows.

An ultrasound imaging probe according to one embodiment includes: a substrate which includes an ultrasonic transducer for detecting an ultrasonic wave formed thereon and a through hole passing through front and rear surfaces; an optical fiber which oscillates a laser (guides/radiates laser light) ; a lens which condenses the laser and is arranged in the through hole; and a tubular housing. The substrate and the optical fiber are fixed to the housing.

A manufacturing method of an ultrasound imaging probe according to one embodiment includes: (a) a process of preparing a substrate which includes an ultrasonic transducer for detecting an ultrasonic wave is formed thereon and a through hole passing through front and rear surfaces; and (b) a process of fixing the substrate to the inside of a tubular housing after the process (a). The manufacturing method of the ultrasound imaging probe further includes (c) a process of arranging a lens within the through hole of the substrate after the process (b) ; and (d) a process of fixing an optical fiber for oscillating a laser to the inside of the housing after the process (c). The substrate and the optical fiber are fixed to the housing.

An ultrasonic imaging device according to one embodiment includes: an ultrasound imaging probe which includes a substrate in which an ultrasonic transducer for detecting an ultrasonic wave is formed thereon, and an optical fiber which oscillates a laser; a laser control part which controls the laser; and a receiving part which receives a signal which is converted from the ultrasonic wave by the ultrasonic transducer. The ultrasonic imaging device further includes: an image processing part which performs image processing on the signal received by the receiving part; and a display part which displays images processed by the image processing part. In the ultrasound imaging probe, the laser is condensed by a lens arranged within a through hole included by the substrate, and the substrate and the optical fiber are fixed to a tubular housing.

### Advantageous Effects of Invention

The effects obtained by representative aspects of the invention disclosed in this application will be briefly described below.

In the ultrasound imaging probe, it is possible to improve the position accuracy of the acoustic element and the optical element to secure the assembly accuracy of the ultrasound imaging probe, and to improve the resolution performance of the obtained image.

### Brief Description of Drawings

[Fig. 1] Fig. 1 is a schematic configuration diagram illustrating one example of an ultrasonic imaging device of an embodiment of the invention.
[Fig. 2] Fig. 2 is a perspective view partially illustrating one example of a use situation of the ultrasonic imaging device of Fig. 1.
[Fig. 3] Fig. 3 is a block diagram illustrating one example of a configuration of the ultrasonic imaging device of Fig. 1.
[Fig. 4] Fig. 4 is a perspective view illustrating one example of a structure of a catheter provided in the ultrasonic imaging device of Fig. 1.
[Fig. 5] Fig. 5 is an enlarged cross-sectional view partially illustrating one example of a structure of a tip part in the catheter of Fig. 4.
[Fig. 6] Fig. 6 is a transparent plan view illustrating one example of a positional relation among members in the catheter of Fig. 5.
[Fig. 7] Fig. 7 is a cross-sectional view partially illustrating one example (without any deviation) of a relation of a positional deviation between a through hole and a lens in the catheter of Fig. 5.
[Fig. 8] Fig. 8 is a cross-sectional view partially illustrating one example (maximum deviation) of the relation of the positional deviation between the through hole and the lens in the catheter of Fig. 5.

### Description of Embodiments

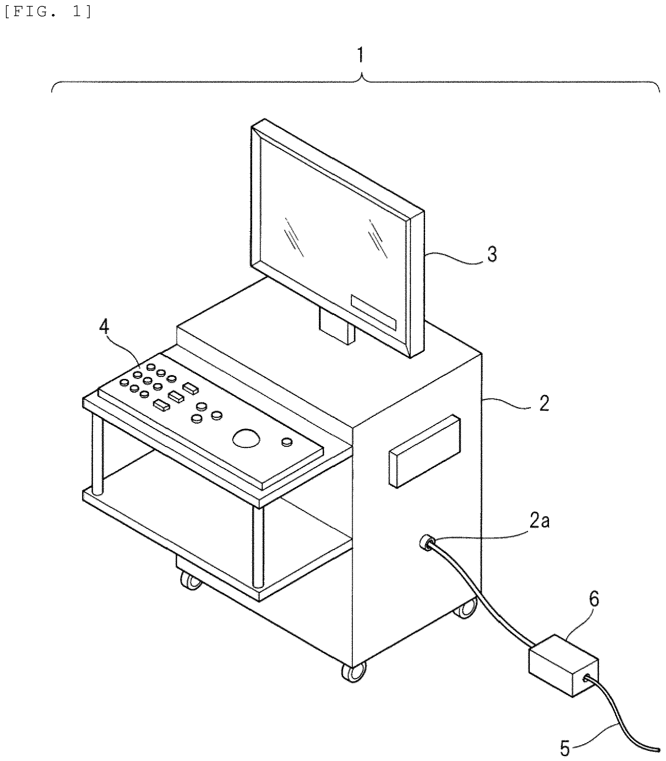

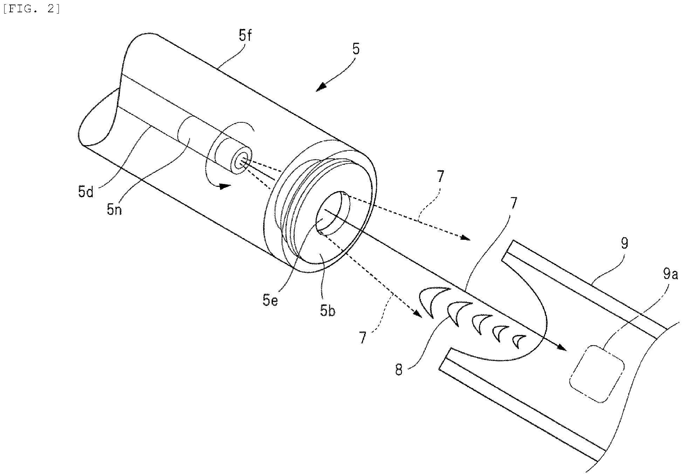

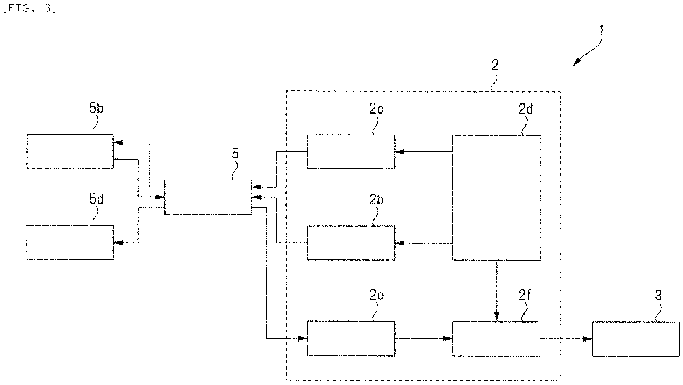

Fig. 1 is a schematic configuration diagram illustrating one example of an ultrasonic imaging device of an embodiment of the invention, Fig. 2 is a perspective view partially illustrating one example of a use situation of the ultrasonic imaging device of Fig. 1, and Fig. 3 is a block diagram illustrating one example of a configuration of the ultrasonic imaging device of Fig. 1.

The ultrasonic imaging device of the embodiment illustrated in Fig. 1 will be described.

An ultrasonic imaging device 1 illustrated in Fig. 1 is an inspection device in which a catheter used in a medical field or the like is attached. For example, a crimped state or the like of a stent and a vascular wall is imaged and inspected by radiating laser on an inspection object part of a blood vessel and detecting an ultrasonic wave which is reflected therefrom.

In the ultrasonic imaging device 1 of the embodiment, the attached catheter is an ultrasound imaging probe which is also called a photoacoustic catheter 5.

The configuration of the ultrasonic imaging device 1 is described using Figs. 1 to 3. The ultrasonic imaging device 1 includes a main body part 2 which includes a laser control part 2b, a reception circuit part (receiving part) 2e, an image processing part 2f, and the like as illustrated in Fig. 3, a catheter connection part 6 which is connected to a connection part 2a of the main body part 2 illustrated in Fig. 1, and a photoacoustic catheter 5 which is an ultrasound imaging probe connected to the catheter connection part 6.

The ultrasonic imaging device 1 includes a display part 3 which projects the image of the inspection object part and an input part 4 which inputs various pieces of information by a key operation at the time of inspecting.

As illustrated in Fig. 2, at the time of inspecting, first, a laser 7 is oscillated from an optical fiber 5d provided in the photoacoustic catheter 5 with respect to the inspection object part, an ultrasonic wave 8 coming out therefrom is detected and received, and the state of the inspection object part is projected to the display part 3. Then, depending on the situation, for example, a treatment is performed such that the laser 7 is radiated on a thrombus 9a of a blood vessel 9 which is an inspection object part to burn off the thrombus 9a.

As illustrated in Figs. 1 to 3, the photoacoustic catheter (ultrasound imaging probe) 5 which is connected to the main body part 2 of the ultrasonic imaging device 1 through the catheter connection part 6 is provided with an ultrasonic transducer (CMUT (Capacitive Micro-machined Ultrasonic Transducers)) 5b and the optical fiber 5d which oscillates the laser 7. The ultrasonic transducer 5b is a Micro Electro Mechanical Systems (MEMS) sensor, and the ultrasonic transducer 5b of the embodiment is, for example, an acoustic element formed in an array type.

On the other hand, the main body part 2 of the ultrasonic imaging device 1 is provided with the laser control part 2b which controls the oscillation of the laser 7 radiated from the optical fiber 5d, a bias part 2c which supplies the bias current to the ultrasonic transducer 5b, the reception circuit part (receiving part) 2e which receives the signal which is converted from the ultrasonic wave 8 detected by the ultrasonic transducer 5b provided in the photoacoustic catheter 5, and the image processing part 2f which performs image processing on the signal received by the reception circuit part 2e.

The main body part 2 is provided with the bias part 2c which supplies the bias current to the ultrasonic transducer 5b and the control part 2d which drives the optical fiber 5d or controls the image processing part 2f.

The display part 3 of the ultrasonic imaging device 1 is a monitor which displays images processed by the image processing part 2f.

Accordingly, in the ultrasonic imaging device 1, it is possible to image the front side of the catheter by the photoacoustic catheter 5.

When the thrombus is imaged and treated by using the ultrasonic imaging device 1, first, the photoacoustic catheter 5 is advanced close to the thrombus under the guidance using an X-ray image. Then, as illustrated in Fig. 2, the optical fiber 5d is driven to be rotated, the laser 7 is radiated on the thrombus 9a, the ultrasonic wave 8 coming out therefrom is detected by the ultrasonic transducer 5b, and the state of the thrombus 9a is projected to the display part 3.

Next, the laser 7 is similarly radiated on the thrombus 9a while checking the image of the display part 3, and the thrombus 9a is burnt off to be removed. Accordingly, the occluded place of the blood vessel 9 is penetrated. Thereafter, the stent is arranged within the blood vessel 9, and the stent is expanded.

The laser 7 is radiated by the photoacoustic catheter 5 to image and check the indwelled state of the stent.

Next, a specific structure of the photoacoustic catheter 5 which is the ultrasound imaging probe of the embodiment will be described.

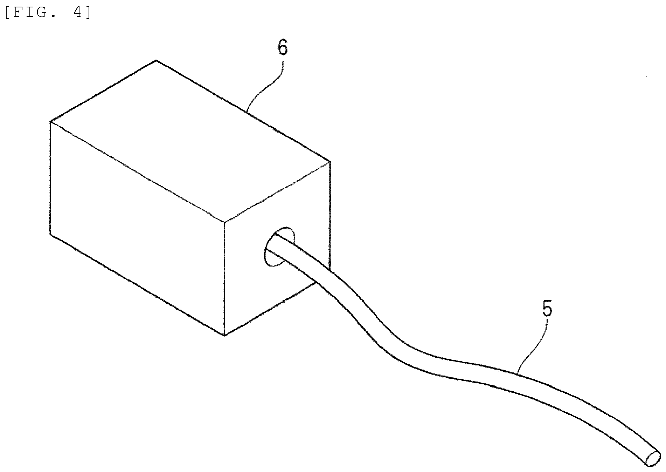

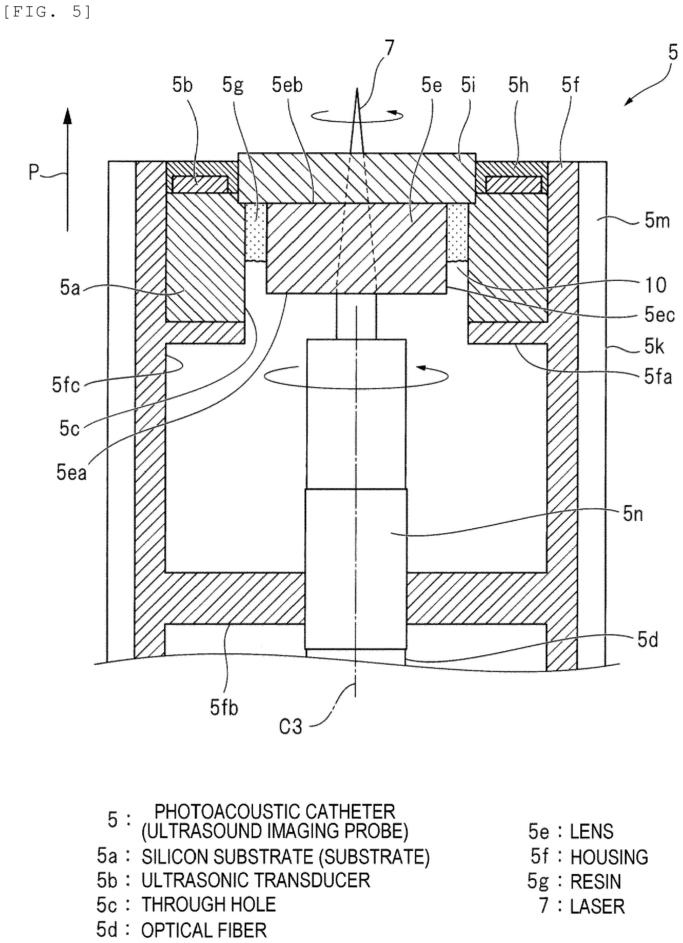

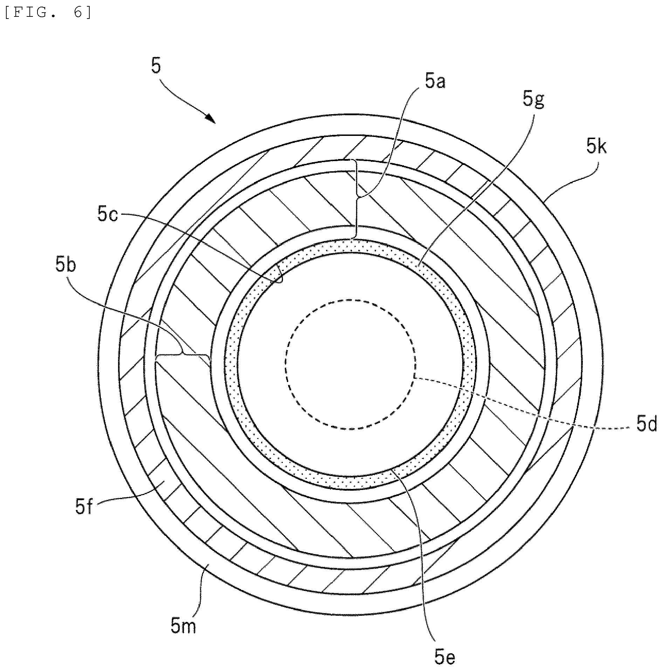

Fig. 4 is a perspective view illustrating one example of the structure of the catheter provided in the ultrasonic imaging device of Fig. 1. Fig. 5 is an enlarged cross-sectional view partially illustrating one example of the structure of the tip part in the catheter of Fig. 4. Fig. 6 is a transparent plan view illustrating one example of a positional relation among members in the catheter of Fig. 5.

As illustrated in Fig. 1, the photoacoustic catheter (ultrasound imaging probe) 5 is connected to the catheter connection part 6 which is connected to the connection part 2a of the main body part 2 of the ultrasonic imaging device 1, and is an elongated tubular member as illustrated in Fig. 4.

As illustrated in Fig. 5, near the tip part of the photoacoustic catheter 5, the ultrasonic transducer 5b for detecting the ultrasonic wave 8 illustrated in Fig. 2 is formed on the surface thereof, and there are provided with a silicon substrate (substrate) 5a including a through hole 5c passing through front and rear surfaces and a lens 5e which condenses the laser 7 and is arranged in the through hole 5c. That is, the silicon substrate 5a and the lens 5e are arranged within the tip part of a housing 5f. The housing 5f has a cylindrical shape (tubular shape) and is an elongated tubular member. In Fig. 5, an electronic circuit substrate or a cable required to transfer the signal of the ultrasonic transducer 5b is omitted. In Fig. 5, a cable for transferring a voltage signal required to drive an actuator 5n is omitted.

The optical fiber 5d which oscillates the laser 7 is stored in the housing 5f in the state of being arranged along the central axis thereof.

In the photoacoustic catheter 5 of the embodiment, the silicon substrate 5a and the optical fiber 5d are fixed to a part of the housing 5f within the housing 5f.

In the silicon substrate 5a, the cylindrical through hole 5c is formed in the central portion, and thus the planar shape thereof is a ring shape and a disc shape. On the other hand, the housing 5f has a cylindrical appearance and also has an almost cylindrical hollow portion therein. Further, the outer circumferential portion of the silicon substrate 5a is fixed to an inner circumferential wall 5fc of the housing 5f.

The ultrasonic transducer 5b is an electrostatic capacitance sensor which is formed on the silicon substrate 5a having a ring shape in plan view and is arranged to surround the through hole 5c in plan view as illustrated in Fig. 6. Further, the cylindrical lens 5e is arranged on the inner circumferential side of the ultrasonic transducer 5b. That is, the cylindrical lens 5e is arranged within the cylindrical through hole 5c of the central portion of the silicon substrate 5a.

A gap 10 between the cylindrical lens 5e and the through hole 5c of the silicon substrate 5a is filled with a transparent resin 5g. Further, the planar shape and the positional relation regarding the housing 5f, the silicon substrate 5a, the ultrasonic transducer 5b, the resin 5g, and the lens 5e are illustrated in Fig. 6.

As illustrated in Fig. 5, the ultrasonic transducer 5b formed on the silicon substrate 5a is covered with a protective film 5h and is protected by the protective film 5h.

The lens 5e has a first surface 5ea which intersects with the oscillating direction P of the laser 7 and a second surface 5eb which is on an opposite side thereto. The second surface 5eb which is positioned on the outside (tip side) between the first surface 5ea and the second surface 5eb is covered with a transparent glass cover 5i which contacts the second surface 5eb. That is, the glass cover 5i has a disc shape and is arranged to block the opening portion on the tip side of the through hole 5c of the silicon substrate 5a.

The ultrasonic transducer 5b is arranged on the circumferential outside of the disc-shaped glass cover 5i, and the circumferential outside is covered with the protective film 5h which fills the position between the glass cover 5i and the housing 5f.

The side surface (third surface) 5ec which is positioned between the first surface 5ea and the second surface 5eb of the lens 5e is partially covered with the transparent resin 5g as described above. Specifically, the above-described gap 10, which is formed in the through hole 5c of the silicon substrate 5a with the inner wall of the through hole 5c of the silicon substrate 5a, the side surface 5ec of the lens 5e, and a part of the glass cover 5i, is filled with the transparent resin 5g.

A resin sheath 5k is provided outside the housing 5f to cover the housing 5f. Accordingly, the area between the sheath 5k and the housing 5f serves as a flow path 5m of a blood removal liquid.

Herein, in the photoacoustic catheter 5 of the embodiment, the silicon substrate 5a and the optical fiber 5d are fixed to the housing 5f. Specifically, the silicon substrate 5a is arranged on a ring-shaped support part 5fa protruding from the inner circumferential wall 5fc toward the center of the housing 5f in the housing 5f, and is fixed to the support part 5fa.

On the other hand, the optical fiber 5d is arranged along the extending direction of the housing 5f in the inner central portion of the cylindrical housing 5f, and is rotatably supported by the support part 5fb protruding from the inner circumferential wall 5fc of the housing 5f.

Accordingly, in the photoacoustic catheter 5 of the embodiment, the silicon substrate 5a and the optical fiber 5d are fixed to the housing 5f. Thus, it is possible to align three axes of the ultrasonic transducer 5b formed on the silicon substrate 5a, the lens 5e arranged in the through hole 5c of the silicon substrate 5a, and the optical fiber 5d.

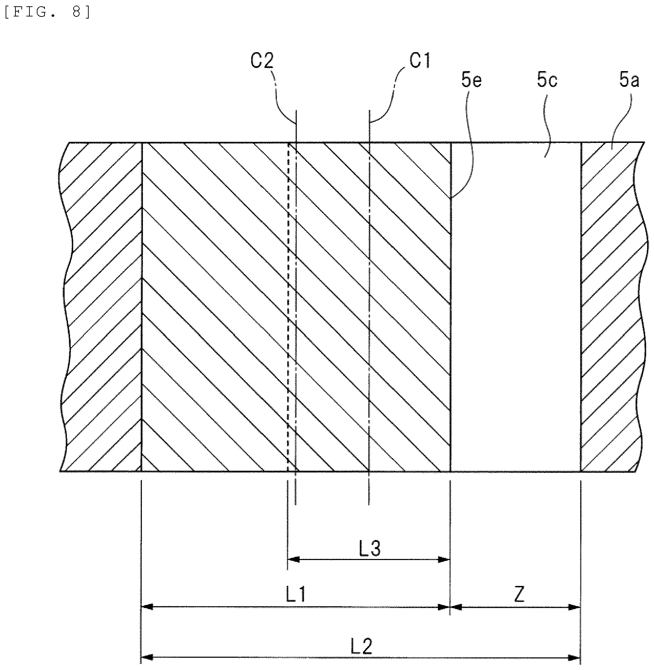

That is, the positioning of the lens 5e is determined by the through hole 5c of the silicon substrate 5a, and the positioning of the silicon substrate 5a and the optical fiber 5d are determined by the housing 5f. Thus, it is possible to align three axes of the ultrasonic transducer 5b, the lens 5e, and the optical fiber 5d. In other words, a center C3 of the optical fiber 5d illustrated in Fig. 5, a center C1 of the silicon substrate 5a illustrated in Fig. 8 which will be described later, and a center C2 of the lens 5e can be matched with a high position accuracy.

As a result, it is possible to secure the assembly accuracy in the photoacoustic catheter (ultrasound imaging probe) 5. In other words, in the photoacoustic catheter 5, it is possible to establish a component mounting method in which the position accuracy of the laser 7 (lens 5e) and the ultrasonic transducer 5b is improved.

For example, in the photoacoustic catheter 5 of the embodiment, it is possible to arrange and fix the acoustic element (ultrasonic transducer 5b) and the optical element (the optical fiber 5d or the lens 5e) with the accuracy of about 100 µm.

In the photoacoustic catheter 5 of the embodiment, the optical fiber 5d is attached such that the tip side thereof is rotatable. Specifically, as illustrated in Fig. 5, the actuator 5n is attached on the tip side of the optical fiber 5d, and the tip side of the optical fiber 5d can be rotated.

Accordingly, it is possible to secure the viewing angle of the laser 7 which is oscillated from the optical fiber 5d.

Herein, for example, preferably, the housing 5f is formed of a metal such as an SUS (stainless steel) or a resin. When the housing 5f is formed of the SUS, it is possible to improve the processing accuracy of the housing 5f since the SUS is a material having a high processing accuracy. It is possible to improve the accuracy of the housing 5f positioning the silicon substrate 5a or the optical fiber 5d.

However, the housing 5f may be formed of a resin on which a fine processing can be performed.

The transparent resin 5g with which the gap 10 between the lens 5e and the through hole 5c of the silicon substrate 5a is filled is, for example, a UV curable resin, a thermosetting resin, or a two-liquid curable resin.

The lens 5e is, for example, a GRIN lens.

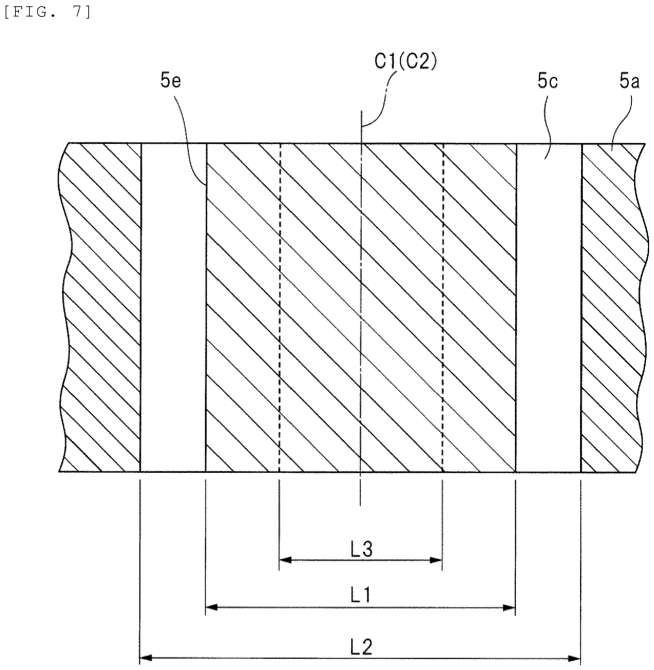

Next, the positional deviation amount between the through hole 5c formed in the silicon substrate 5a and the lens 5e will be described. Fig. 7 is a cross-sectional view partially illustrating one example (without any deviation) of the relation of the positional deviation between the through hole and the lens in the catheter of Fig. 5. Fig. 8 is a cross-sectional view partially illustrating one example (maximum deviation) of the relation of the positional deviation between the through hole and the lens in the catheter of Fig. 5.

It is ideal that the position of the lens 5e with respect to the through hole 5c of the silicon substrate 5a is the position as illustrated in Fig. 7. That is, the lens 5e is arranged in the center of the through hole 5c. Herein, the center C1 of the through hole 5c is arranged to match the center C2 of the lens 5e when the diameter L1 of the lens 5e is 350 µm, the diameter L2 of the through hole 5c is 500 µm, and the range L3 of the laser scan is 200 µm. Further, since the range L3 of the laser scan is 200 µm, the laser scan can be performed on the vicinity of the center with respect to 350 µm of the diameter L1 of the lens 5e.

Fig. 8 is the case where the center C2 of the lens 5e is arranged to be maximally deflected to the left side with respect to the center C1 of the through hole 5c (a case where an allowable deviation amount is maximum). In this case, the end of the range L3 of the laser scan is overlapped with the end of the range of the diameter L1 of the lens 5e, and the range L3 of the laser scan indicates a range where the positional deviation of the lens 5e is maximum when the range is set not to be out of the lens.

In the positional deviation of the lens 5e with respect to the through hole 5c of the silicon substrate 5a, it is important to keep the range L3 of the laser scan from being out of the lens. If the range L3 of the laser scan is out of the lens, the power of the laser 7 is reduced, the desired photoacoustic signal is not generated, and the quality of the captured image is lowered due to the sensitivity deficiency. At that time, the deviation amount of the lens 5e with respect to the through hole 5c is associated with the range L3 of the laser scan and the diameter L1 of the lens 5e.

Therefore, a case where the deviation amount of the lens 5e is maximum as illustrated in Fig. 8 is the limit of the allowable range. In the structure illustrated in Fig. 8, a difference Z between the diameter L2 of the through hole 5c of the silicon substrate 5a and the diameter L1 of the lens 5e is 150 µm, and the limit value of the numerical value of the difference Z is 150 µm. That is, the difference Z between the diameter L2 of the through hole 5c of the silicon substrate 5a and the diameter L1 of the lens 5e is preferably within 150 µm.

The difference Z between the diameter L2 of the through hole 5c and the diameter L1 of the lens 5e is within 150 µm as described above, thereby avoiding the reduction of the power of the laser 7 and preventing the lowering of the quality of the captured image.

The ultrasonic imaging device 1 illustrated in Fig. 1 according to the embodiment includes the above-described photoacoustic catheter (ultrasound imaging probe) 5 illustrated in Fig. 5. That is, in the above-described photoacoustic catheter 5, the laser 7 is condensed by the lens 5e which is arranged within the through hole 5c included in the silicon substrate 5a, and the silicon substrate 5a (ultrasonic transducer 5b) and the optical fiber 5d are respectively fixed to a part of the housing 5f inside the tubular housing 5f.

According to the ultrasonic imaging device 1 of the embodiment, in the photoacoustic catheter 5 included in the ultrasonic imaging device 1, the lens 5e is arranged in the through hole 5c of the silicon substrate 5a, and the silicon substrate 5a and the optical fiber 5d are fixed to the housing 5f, thereby improving the position accuracy of the ultrasonic transducer 5b (acoustic element) on the silicon substrate 5a and an optical element such as the optical fiber 5d or the lens 5e.

As a result, it is possible to improve the acoustic performance of the ultrasonic imaging device 1. Specifically, in the ultrasonic imaging device 1, the assembly accuracy of the photoacoustic catheter 5 included in the ultrasonic imaging device 1 can be secured. Accordingly, in the ultrasonic imaging device 1, the resolution performance of the obtained image can be improved.

The ultrasonic imaging device 1 of the embodiment includes the photoacoustic catheter 5. Thus, the thrombus can be removed mainly with respect to chronic total occlusion lesion (CTO) by the front-side imaging of the catheter and the laser radiation with high power. As a result, it is possible to perform the stent treatment of the CTO which is considered to be difficult.

Next, the manufacturing method of the photoacoustic catheter (ultrasound imaging probe) 5 of the embodiment will be described. In the embodiment, described is a case in which the photoacoustic catheter 5 is manufactured by using the manufacturing process of the semiconductor process.

First, as illustrated in Fig. 5, the electrostatic capacitance ultrasonic transducer 5b is formed on the silicon substrate 5a by using the semiconductor process. That is, on the silicon substrate 5a, the ultrasonic transducer 5b which is an electrostatic capacitance type and an MEMS sensor is formed to surround the through hole 5c in plan view.

Next, the through hole 5c which passes through the front and rear surfaces is formed in the central portion of the silicon substrate 5a. Here, for example, the through hole 5c is formed in the substantially central portion of the silicon substrate 5a by etching processing.

That is, the ultrasonic transducer (CMUT) 5b which detects the ultrasonic wave is formed on the surface, and the silicon substrate 5a which includes the through hole 5c passing through the front and rear surfaces is prepared on the inner circumferential side of the ultrasonic transducer 5b. Further, the elongated housing 5f having a tubular shape is prepared. The outer circumferential shape (circular shape) of the silicon substrate 5a is also formed by the etching processing at the time of forming the through hole 5c. That is, the silicon substrate 5a is formed in a disc shape by the same etching processing with the through hole 5c. Accordingly, the silicon substrate 5a also has a ring shape (disc shape) in plan view.

However, each of the through hole 5c and the outer circumferential shape of the silicon substrate 5a may be processed by separate processes.

Next, the silicon substrate 5a in which the ultrasonic transducer 5b is formed thereon is fixed to the inside of the tubular housing 5f. Herein, in the housing 5f, the silicon substrate 5a is fixed to the support part 5fa protruding from the inner circumferential wall 5fc of the housing 5f. At that time, the outer circumferential portion of the silicon substrate 5a is fixed to the inner circumferential wall 5fc of the housing 5f, and is arranged on the support part 5fa. Accordingly, the silicon substrate 5a and the ultrasonic transducer 5b are positioned by the inner circumferential wall 5fc of the housing 5f.

Next, the transparent glass cover 5i is attached to the silicon substrate 5a. At that time, on the inner circumferential side of the ultrasonic transducer 5b formed on the silicon substrate 5a, the disc-shaped glass cover 5i is attached to the silicon substrate 5a to block the opening portion on the tip side of the through hole 5c of the silicon substrate 5a.

Accordingly, in plan view, the ultrasonic transducer 5b is arranged between the housing 5f and the glass cover 5i.

Next, the area between the glass cover 5i and the housing 5f is filled with the protective film 5h which protects the ultrasonic transducer 5b.

Next, both sides of the housing 5f are reversed, and the cylindrical lens 5e is arranged within the through hole 5c of the silicon substrate 5a in the state (a state where the opening portion of the through hole 5c of the silicon substrate 5a is directed upward).

At that time, the lens 5e is fitted into the through hole 5cto contact the second surface 5eb of the lens 5e with the glass cover 5i. Further, after the lens 5e is arranged in the through hole 5c of the silicon substrate 5a, the gap 10 between the lens 5e and the through hole 5c is filled with the transparent resin 5g.

Specifically, the transparent resin 5g is poured into the gap 10 formed by the cylindrical lens 5e, the inner wall of the through hole 5c of the silicon substrate 5a, and the glass cover 5i with the disc shape to firmly fix the lens 5e.

Accordingly, the cylindrical lens 5e is positioned by the through hole 5c of the silicon substrate 5a.

Next, the optical fiber 5d which oscillates the laser 7 is fixed inside the housing 5f. Specifically, in the housing 5f, the optical fiber 5d is fixed to the support part 5fb which protrudes from the inner circumferential wall 5fc of the housing 5f. At that time, the fixing position of the optical fiber 5d is adjusted such that the center C1 of the disc-shaped silicon substrate 5a illustrated in Fig. 8 matches the center C3 of the optical fiber 5d illustrated in Fig. 5. For example, the adjusting method of the optical fiber 5d may be performed such that the laser for adjustment is radiated from the optical fiber 5d, and the laser is radiated on a predetermined position. For example, the optical fiber 5d is easily adjusted when visible light such as red laser is used as the laser for adjustment.

When the fixing position of the optical fiber 5d is adjusted, the actuator 5n may be driven to check whether the laser 7 radiated from the optical fiber 5d is rotated within a predetermined range.

Next, the outer circumferential portion of the housing 5f is covered with the resin sheath 5k. Accordingly, the photoacoustic catheter 5 in which the silicon substrate 5a and the optical fiber 5d are fixed to the housing 5f is assembled completely.

In the assembly of the photoacoustic catheter 5 of the embodiment, by the etching processing of the semiconductor manufacturing process, the through hole 5c of the silicon substrate 5a is formed, and the outer circumferential portion of the silicon substrate 5a is formed in a circular shape. Therefore, it is possible to improve the processing accuracy of the through hole 5c and the inner circumference of the substrate.

Accordingly, it is possible to improve the positioning accuracy of the lens 5e arranged within the through hole 5c or the positioning accuracy of the silicon substrate 5a attached to the inside of the housing 5f. As a result, it is possible to improve the position accuracy of the lens 5e and the ultrasonic transducer 5b on the silicon substrate 5a. That is, it is possible to improve the assembly accuracy of the photoacoustic catheter 5, and it is possible to improve the resolution performance of the obtained image in the ultrasonic imaging device 1 using the photoacoustic catheter 5.

In the photoacoustic catheter 5 of the embodiment, since the optical fiber 5d is rotatable, it is possible to rotate and radiate the laser 7 and to widen the angle of the visual field as compared to the probe which performs the ultrasonic wave radiation.

Since the ultrasonic transducer 5b is formed on the silicon substrate 5a by the semiconductor manufacturing process, the ultrasonic transducer 5b can be formed on the silicon substrate 5a with a high position accuracy.

Hereinbefore, the invention made by the present inventors has been described in detail based on the embodiment. However, the invention is not limited to the above-described embodiment and includes various modifications. For example, the above-described embodiment is intended to be illustrative of the invention in an easily understandable manner, and the invention is not necessarily limited to the one that includes all of the components described in the embodiment.

Some of a configuration of one embodiment can be substituted by the configuration of another embodiment. In addition, the configuration of the another embodiment can be added to the configuration of the one embodiment. Also, in some of the configuration of each embodiment, addition of another configuration, deletion and substitution are possible. Each member or a relative size in the drawings is simplified and idealized for explaining the invention in an easily understandable manner, and has more complicated shapes when mounted.

In the embodiment, the description is given about a case where the optical fiber 5d is fixed to the support part 5fb of the housing 5f. However, the silicon substrate 5a may be formed to be thick by sticking the silicon substrates 5a, and the optical fiber 5d may be supported in the structure which is formed by the assembly using the semiconductor manufacturing process.

### Reference Signs List

- 1:: ultrasonic imaging device
- 2:: main body part
- 2a:: connection part
- 2b:: laser control part
- 2c:: bias part
- 2d:: control part
- 2e:: reception circuit part (receiving part)
- 2f:: image processing part
- 3:: display part
- 4:: input part
- 5:: photoacoustic catheter (ultrasound imaging probe)
- 5a:: silicon substrate (substrate)
- 5b:: ultrasonic transducer
- 5c:: through hole
- 5d:: optical fiber
- 5e:: lens
- 5ea:: first surface
- 5eb:: second surface
- 5ec:: side surface (third surface)
- 5f:: housing
- 5fa:: support part
- 5fb:: support part
- 5fc:: inner circumferential wall
- 5g:: resin
- 5h:: protective film
- 5i:: glass cover
- 5k:: sheath
- 5m:: flow path
- 5n:: actuator
- 7:: laser
- 8:: ultrasonic wave
- 9:: blood vessel
- 9a:: thrombus
- 10:: gap

## Claims

1. An ultrasound imaging probe comprising:
a substrate which includes an ultrasonic transducer for detecting an ultrasonic wave formed thereon and a through hole passing through front and rear surfaces;
an optical fiber which oscillates a laser;
a lens which condenses the laser and is arranged in the through hole; and
a tubular housing, wherein
the substrate and the optical fiber are fixed to the housing.

2. The ultrasound imaging probe according to claim 1, wherein
the lens includes a first surface which intersects with an oscillating direction of the laser and a second surface which is on an opposite side to the first surface,
the second surface which is positioned on an outer side among the first surface and the second surface is covered with a glass cover which contacts the second surface, and
a third surface which is positioned between the first surface and the second surface is covered with a resin.

3. The ultrasound imaging probe according to claim 1 or 2, wherein
each of the lens and the through hole has a cylindrical shape, and
a difference between a diameter of the through hole and a diameter of the lens is within 150 µm.

4. The ultrasound imaging probe according to any of claims 1 to 3, wherein
the ultrasonic transducer is an electrostatic capacitance sensor formed on a silicon substrate and is arranged around the through hole in plan view, and
the lens having a cylindrical shape is arranged on an inner circumferential side of the ultrasonic transducer.

5. The ultrasound imaging probe according to any of claims 1 to 4, wherein
the substrate has a disc shape,
the housing has a cylindrical shape, and
an outer circumferential portion of the substrate contacts an inner circumferential wall of the housing.

6. A manufacturing method of an ultrasound imaging probe comprising:
(a) a process of preparing a substrate which includes an ultrasonic transducer for detecting an ultrasonic wave formed thereon and a through hole passing through front and rear surfaces;
(b) a process of fixing the substrate to the inside of a tubular housing after the process (a);
(c) a process of arranging a lens within the through hole of the substrate after the process (b); and,
(d) a process of fixing an optical fiber for oscillating a laser to the inside of the housing after the process (c), wherein
the substrate and the optical fiber are fixed to the housing.

7. The manufacturing method of the ultrasound imaging probe according to claim 6, wherein
the process (a) has
(a1) a process of forming the ultrasonic transducer which is an electrostatic capacitance type on a silicon substrate which is the substrate, and
(a2) a process of forming the through hole passing through the front and rear surfaces in the silicon substrate after the process (a1).

8. The manufacturing method of the ultrasound imaging probe according to claim 7, wherein
in the process (a2), the through hole is formed by an etching processing.

9. The manufacturing method of the ultrasound imaging probe according to any of claims 6 to 8, wherein
in the process (c), the lens is fitted into the through hole, and a gap between the lens and the through hole is filled with a resin.

10. An ultrasonic imaging device comprising:
an ultrasound imaging probe which includes a substrate in which an ultrasonic transducer for detecting an ultrasonic wave is formed thereon, and an optical fiber which oscillates a laser;
a laser control part which controls the laser;
a receiving part which receives a signal which is converted from the ultrasonic wave by the ultrasonic transducer;
an image processing part which performs image processing on the signal received by the receiving part; and
a display part which displays images processed by the image processing part, wherein
in the ultrasound imaging probe, the laser is condensed by a lens arranged within a through hole included by the substrate, and the substrate and the optical fiber are fixed to a tubular housing.

11. The ultrasonic imaging device according to claim 10, wherein
the lens provided in the ultrasound imaging probe includes a first surface which intersects with an oscillating direction of the laser and a second surface which is on an opposite side to the first surface,
the second surface which is positioned on an outer side among the first surface and the second surface is covered with a glass cover which contacts the second surface, and
a third surface which is positioned between the first surface and the second surface is covered with a resin.

12. The ultrasonic imaging device according to claim 10 or 11, wherein
each of the lens and the through hole included by the substrate, which are provided in the ultrasound imaging probe, has a cylindrical shape, and
a difference between a diameter of the through hole and a diameter of the lens is within 150 µm.

13. The ultrasonic imaging device according to any of claims 10 to 12, wherein
the ultrasonic transducer included in the ultrasound imaging probe is an electrostatic capacitance sensor formed on a silicon substrate and is arranged around the through hole in plan view, and
the lens having a cylindrical shape is arranged on an inner circumferential side of the ultrasonic transducer.

14. The ultrasonic imaging device according to any of claims 10 to 13, wherein
the substrate provided in the ultrasound imaging probe has a disc shape,
the housing has a cylindrical shape, and
an outer circumferential portion of the substrate is fixed to an inner circumferential wall of the housing.
